# EUROPEAN PATENT APPLICATION

(11) **EP 2 332 932 A1**
(43) Date of publication of application: **15.06.2011**
(21) Application number: 09813084.2
(22) Date of filing: 09.09.2009
(51) Int. Cl.: C07D 401/14, A61K 31/501, A61P 19/02, A61P 29/00, A61P 43/00

(54) **NOVEL CRYSTAL FORMS OF 4-(2-AMINOPYRIDIN-4-YL)-3-(4 FLUORO- PHENYL)-1-(1,4,5,6-TETRAHYDRO-6-OXOPYRIDAZIN-3-YL)-1H-PYRA-ZOLE METHANESULFONATE AND PROCESS FOR PRODUCTION OF SAME**

(30) Priority: 09.09.2008 JP 2008230898
(71) Applicant: Ube Industries, Ltd., Ube-shi Yamaguchi 755-8633 (JP)
(72) Inventor: SHIMIZU, Motohisa, Yamaguchi 755-8633 (JP); SUZUKI, Takahiro, Yamaguchi 755-8633 (JP); MOTOYAMA, Takahiro, Yamaguchi 755-8633 (JP); MIYATA, Hiroyuki, Yamaguchi 755-8633 (JP)
(74) Representative: HOFFMANN EITLE
(86) International application number: PCT/JP2009/065728
(87) International publication number: WO 2010/029938

(57) **Abstract**

The present inventions are directed to novel crystal forms of 4-(2-aminopyridin-4-yl)-3 -(4-fluorophenyl)-1-(1,4,5,6-tetrahydro-6-oxopyridazin-3-yl)-1H-pyrazole methanesulfonate, which is useful as a pharmaceutical product, and methods for their preparation.

The present inventions provide two stable crystalline polymorphs called A-form crystal and B-form crystal as well as methods for their preparation, by which each crystalline polymorph can be individually obtained as a single crystal form.

## Description

### Field of the Invention

The present invention relates to novel crystal forms of 4-(2-aminopyridin-4-yl)-3-(4-fluorophenyl)-1-(1,4,5,6-tetrahydro-6-oxopyridazin-3-yl)-1H-pyrazole methanesulfonate and methods for their preparation.

### Background Art

4-(2-Aminopyridin-4-yl)-3-(4-fluorophenyl)-1-(1,4,5,6-tetrahydro-6-oxopyridazin-3-yl)-1H-pyrazole methanesulfonate (hereinafter, abbreviated to "Compound (I)") is a compound useful as a pharmaceutical product, and disclosed in, for example, Patent document 1.

Patent document 1 discloses Compound (I) in Example 80. It also discloses that Compound (I) has excellent inhibitory action of p38MAP kinase and excellent suppressive effects on inflammatory cytokine production (particularly, TNF-α or IL-6 production) based thereon and is useful as a prophylactic or therapeutic agent for diseases in which inflammatory cytokines are implicated (e.g., chronic rheumatoid arthritis, osteoarthritis, an allergic disease, sepsis, psoriasis, osteoporosis, ulcerative colitis, diabetes, or arteriosclerosis). However, there is neither description nor teaching about the presence of crystalline polymorph of Compound (I).

Patent document 1: WO2004/029043 pamphlet

### Disclosure of the Invention

### Problems to be Solved by the Invention

We have made studies on Compound (I) and, during the studies, we have found that Compound (I) prepared by the method described in Patent document 1 does not have a single crystal form but has two or more crystal forms mixed, and that the ratio of the mixed crystal forms varies depending on the conditions for the preparation of crystal, so that the control of the quality of the crystal is very difficult.

Generally, a compound having crystalline polymorphs may exhibit different properties for each crystal form. Especially in the field of pharmaceutical products, it is known that the change of the crystal form results in a difference in, for example, solubility, dissolution speed, stability, or absorption. For this reason, it is considered that even when the same compound is used, the difference of the crystal form makes it impossible to obtain a desired intensity of the action or causes an unexpected intensity of the action, leading to an unforeseen accident. Therefore, it is desired to provide a compound with the constant quality which can be expected to always exhibit a constant intensity of the action.

Compound (I) has crystalline polymorphs as mentioned above, and further the change of the method for preparing the compound causes a difference in the ratio of the crystal forms, and thus even a compound of the constant quality cannot be obtained. Therefore, for achieving a constant effect of action and constant quality, which are the essential requirement of a pharmaceutical product, it has been strongly desired to find a stable crystal form of this compound and further to establish a method for constantly obtaining the stable crystalline polymorph.

### Means to Solve the Problems

We have made extensive studies on the crystal of Compound (I) which is useful as a prophylactic or therapeutic agent for diseases in which inflammatory cytokines are implicated. As a result, it has been found that two crystalline polymorphs, i.e., an A-form crystal and a B-form crystal of the Compound (I) are present, and that the A-form crystal and B-form crystal can be individually prepared in the single crystal form, and thereby the present inventions have been completed.

The present invention is directed to the following:
(I) a substantially pure crystal of 4-(2-aminopyridin-4-yl)-3-(4-fluorophenyl)-1-(1,4,5,6-tetrahydro-6-oxopyridazin-3-yl)-1H-pyrazole methanesulfonate represented by the following general formula (I):

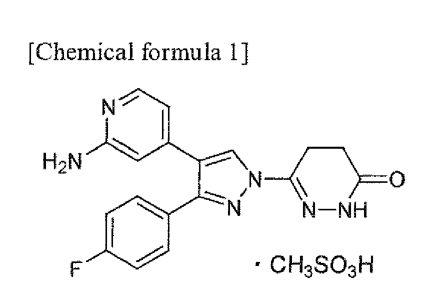

(2) the crystal (A-form crystal) according to item (1) above, which shows main d spacing peaks at 8.5, 5.7, 4.9, and 4.2 angstroms in a powder X-ray diffraction pattern obtained by irradiation with Cu-Kα rays,
(3) the crystal (A-form crystal) according to item (1), which shows diffraction angles 2θ at 10.4±0.2, 15.7±0.2, 1.8.2±0.2, and 21.0±0.2 (degrees) in a powder X-ray diffraction pattern obtained by irradiation with Cu-Kα rays,
(4) a method for preparing the A-form crystal according to item (2) or (3) above, which comprises reacting 4-(2-aminopyridin-4-yl)-3-(4-fluorophenyl)-1-(1,4,5,6-tetrahydro-6-oxopyridazin-3-yl)-1H-pyrazole with methanesulfonic acid in a good solvent, and then adding a poor solvent portionwise and stirring the resultant mixture while cooling,
(5) the method according to item (4) above, wherein the temperature at the start of the first addition of the poor solvent in the portionwise addition of the poor solvent is 20 to 60°C,
(6) the method according to item (4) above, wherein the temperature for the stirring of the mixture while cooling is 0 to 25°C,
(7) the method according to item (4) above, which comprises, after stirring the mixture while cooling, further dropping or adding the poor solvent,
(8) the method according to item (4) above, wherein the good solvent is dimethyl sulfoxide,
(9) the method according to item (4) above, wherein the poor solvent is ethyl acetate,
(10) a method for preparing the A-form crystal according to item (2) or (3) above, which comprises recrystallizing 4-(2-aminopyridin-4-yl)-3-(4-fluorophenyl)-1-(1,4,5,6-tetrahydro-6-oxapyridazin-3-yl)-1H-pyrazole methanesulfonate by dissolving in a good solvent, and then adding a poor solvent and a seed crystal of the A-form crystal,
(11) the method according to item (10) above, wherein the crystallization temperature in the recrystallization is 20 to 60°C,
(12) the method according to item (10) above, wherein the good solvent is dimethyl sulfoxide,
(13) the method according to item (10) above, wherein the poor solvent is ethyl acetate,
(14) the crystal (B-form crystal) according to item (1) above, which shows main d spacing peaks at 10.9, 5.4, 5.2, and 4.7 angstroms in a powder X-ray diffraction pattern obtained by irradiation with Cu-Kα rays,
(15) the crystal (B-form crystal) according to item (1) above, which shows diffraction angles 2θ at 8.1±0.2, 16.3±0.2, 17.0±0.2, and 18.9±0.2 (degrees) in a powder X-ray diffraction pattern obtained by irradiation with Cu-Kα rays,
(16) a method for preparing the B-form crystal according to item (14) or (15) above, which comprises performing solid phase transition from an A-form crystal of 4-(2-aminopyridin-4-yl)-3-(4-fluorophenyl)-1-(1,4,5,6-tetrahydro-6-oxopyridazin-3-yl)-1H-pyrazole methanesulfonate,
(17) the method according to item (16) above, wherein the solid phase transition is performed at 270 to 280°C,
(18) a method for preparing the B-form crystal according to item (14) or (15) above, which comprises recrystallizing 4-(2-aminopyridin-4-yl)-3-(4-fluorophenyl)-1-(1,4,5,6-tetrahydro-6-oxopyridazin-3-yl)-1H-pyrazole methanesulfonate by dissolving in a good solvent, and then adding a poor solvent and a seed crystal of the B-form crystal,
(19) the method according to item (18) above, wherein the crystallization temperature in the recrystallization is 40 to 80°C,
(20) the method according to item (18) above, wherein the good solvent is dimethyl sulfoxide,
(21) the method according to item (18) above, wherein the poor solvent is ethyl acetate,
(22) a method for preparing the A-form crystal according to item (2) or (3) above, which comprises performing solvent-mediated phase transition from a B-form crystal of 4-(2-aminopyridin-4-yl)-3-(4-fluorophenyl)-1-(1,4,5,6-tetrahydro-6-oxopyridazin-3-yl)-1H-pyrazole methane sulfonate,
(23) the method according to item (22) above, wherein the solvent-mediated phase transition is a mixed solvent of dimethyl sulfoxide and ethyl acetate,
(24) the method according to item (22) above, which comprises adding a seed crystal of the A-form crystal,
(25) a pharmaceutical composition comprising the crystal of 4-(2-aminopyridin-4-yl)-3-(4-fluorophenyl)-1-(1,4,5,6-tetrahydro-6-oxopyridazin-3-yl)-1H-pyrazole methanesulfonate according to any one of items (1), (2), (3), (14), and (15) above as an active ingredient,
(26) a p38MAP kinase inhibitor comprising the crystal of 4-(2-aminopyridin-4-yl)-3-(4-fluorophenyl)-1-(1,4,5,6-tetrahydro-6-oxopyridazin-3-yl)-1H-pyrazole methanesulfonate according to any one of items (1), (2), (3), (14), and (15) above as an active ingredient, and
(27) a therapeutic agent for rheumatic diseases comprising the crystal of 4-(2-aminopyridin-4-yl)-3-(4-fluorophenyl)-1-(1,4,5,6-tetrahydro-6-oxopyridazin-3-yl)-1H-pyrazole methanesulfonate according to any one of items (1), (2), (3), (14), and (15) above as an active ingredient.

When the crystal of Compound (I) of the present invention is allowed to stand in the air or subjected to recrystallization, the crystal may absorb water, have adsorbed water attached thereto, or form a hydrate thereof. Such crystal of Compound (I) containing water is included in the present invention. Further, a crystal of a solvate of Compound (I), which contains an arbitrary amount of a solvent, is also included in the present invention.

In the present invention, the crystal of Compound (I) may have an amorphous solid mixed thereinto, and such a mixture of the crystal and amorphous solid in an arbitrary ratio is also included in the present invention. Specifically, the crystal of the present invention preferably contains 50% or more of a crystalline substance, more preferably 80% or more, further preferably 90% or more, especially preferably 95% or more, most preferably 97% or more of a crystalline substance.

In the present invention, the crystal (crystalline substance) represents a solid having an internal structure three-dimensionally formed by regular repetition of constituent atoms (or groups thereof), and is distinguished from an amorphous solid not having such a regular internal structure. Whether or not a solid is a crystal can be examined by a crystallographically known method (e.g., powder X-ray crystallography or differential scanning calorimetry). For example, in powder X-ray crystallography of a solid using X-rays obtained by irradiation with Cu-Kα rays, the solid is determined to be a crystal when a specific peak is observed in its X-ray diffraction pattern, and the solid is determined to be amorphous when a specific peak is not observed. The solid is determined to be a crystal having a low degree of crystallinity when the peak can be read but is not clear (for example, broad). Such a crystal having a low degree of crystallinity can also be included in the crystal of the present invention.

In powder X-ray crystallography using Cu-Kα rays, a sample is usually irradiated with Cu-Kα rays (in which Kα1 and Kα2 rays are not separated). An X-ray diffraction pattern can be obtained by analyzing diffraction derived from Kα rays, or alternatively can be obtained by analyzing only diffraction derived from Kα1 rays taken from diffraction derived from Kα rays. In the present invention, the powder X-ray diffraction pattern obtained by irradiation with Kα rays includes an X-ray diffraction pattern obtained by analyzing diffraction derived from Kα rays and an X-ray diffraction pattern obtained by analyzing diffraction derived from Kα1 rays and is preferably an X-ray diffraction pattern obtained by analyzing diffraction derived from Kα1 rays.

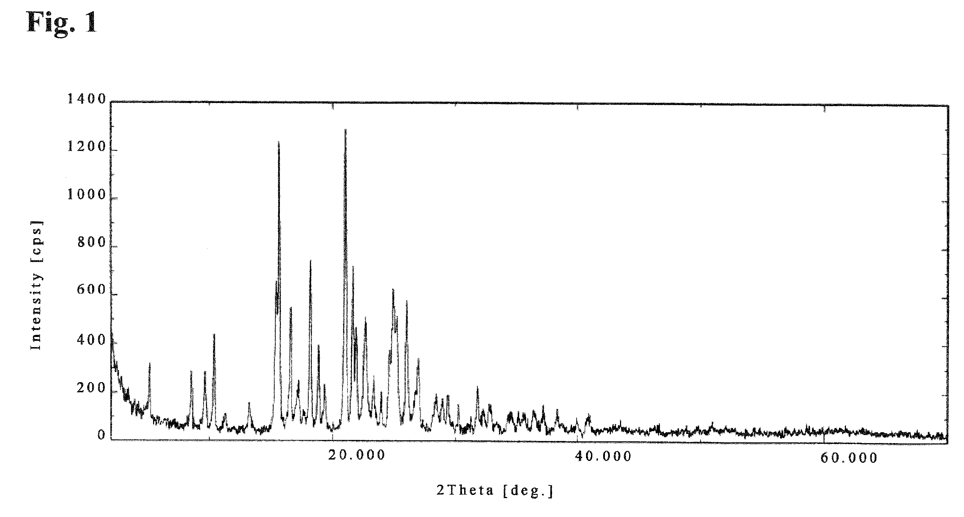

The A-form crystal of 4-(2-aminopyridin-4-yl)-3-(4-fluorophenyl)-1-(1,4,5,6-tetrahydro-6-oxopyridazin-3-yl)-1H-pyrazole methanesulfonate of the present invention is a crystal showing main d spacing peaks at 8.5, 5.7, 4.9, and 4.2 angstroms in a powder X-ray diffraction pattern obtained by irradiation with Cu-Kα rays {namely, diffraction angles 2θ at 10.4, 15.7, 18.2, and 21.0 (degrees)}, for example, as shown in Fig. 1, 4, or 5.

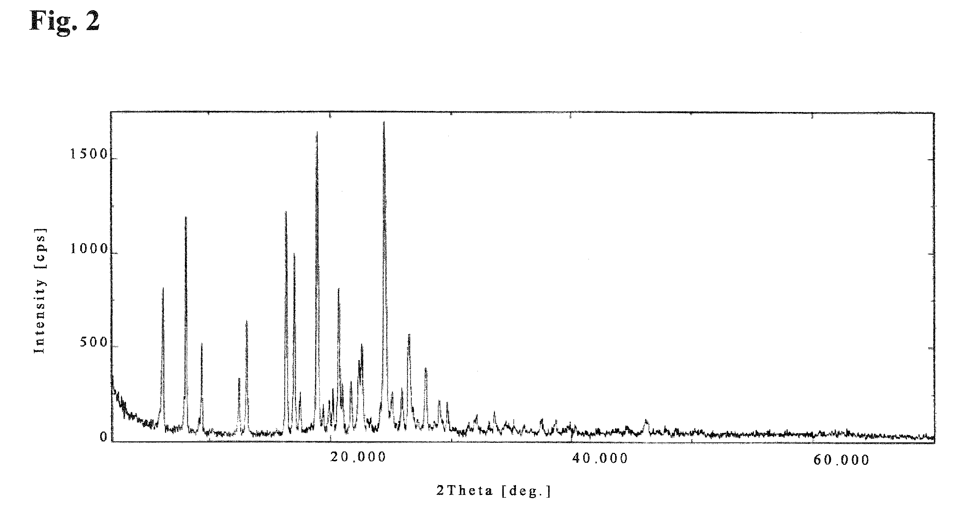

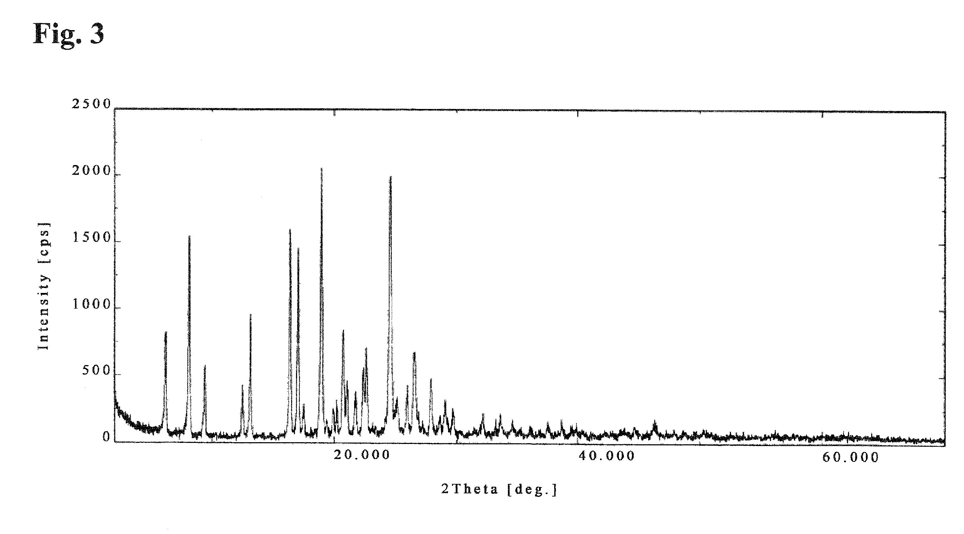

The B-form crystal of 4-(2-aminopyridin-4-yl)-3-(4-fluorophenyl)-1-(1,4,5,6-tetrahydro-6-oxopyridazin-3-yl)-1H-pyrazole methanesulfonate of the present invention is a crystal showing main d spacing peaks at 10.9, 5.4, 5.2, and 4.7 angstroms in a powder X-ray diffraction pattern obtained by irradiation with Cu-Kα rays {namely, diffraction angles 2θ at 8.1, 16.3, 17.0, and 18.9 (degrees)}, for example, as shown in Fig. 2 or 3.

In the powder X-ray diffraction patterns of Figs. 1 to 5 below, the ordinate axis indicates a diffraction intensity [count/second (cps)], and the abscissa axis indicates a diffraction angle 2θ (degree). The d spacing (angstrom) can be calculated by the formula: 2dsinθ = nλ where n = 1. In this formula, Kα rays have a wavelength λ of 1.54 angstrom, and Kα1 rays have a wavelength λ of 1.541 angstrom. Diffraction angle 2θ can be slightly changed in the position and relative intensity depending on the measurement conditions and the like, therefore even when the value of diffraction angle 2θ is slightly changed (±0.2), the identification of the crystal form should be appropriately determined with reference to the pattern of the whole spectrum. This applies to the slight change of the value of the d spacing.

The term "substantially pure" used in the present specification is used when the purity of an A-form or B-form crystal phase is referred. In the present invention, the substantially pure crystal means a crystal such that an A-form crystal or a B-form crystal constitutes 90% or more of the whole crystalline substance, preferably means a crystal such that an A-form crystal or a B-form crystal constitutes 95% or more of the whole crystalline substance.

### Effect of the Invention

In the past, 4-(2-aminopyridin-4-yl)-3-(4-fluorophenyl)-1-(1,4,5,6-tetrahydro-6-oxopyridazin-3-yl)-1H-pyrazole methanesulfonate {Compound (I)} has been able to be obtained only in the form of mixtures of the A-form and B-form crystals in various ratios. Now, the present invention provides a substantially pure A-form crystal or B-form crystal. The A-form crystal and B-form crystal of Compound (I) of the present invention are advantageous not only in that they have excellent properties with respect to the physical properties for drug substance including solubility and dissolution speed, the chemical stability, and the powder physical properties including fluidity and compressibility, but also in that they can exhibit stable pharmacokinetics and beneficial effect profile when administered to a warm-blooded animal. Further, the present inventions also provide methods for preparing the above crystals with ease and excellent reproducibility.

### Brief Description of the Drawings

[Fig. 1] Fig. 1 shows a powder X-ray diffraction pattern of the A-form crystal prepared in Example 1, obtained by irradiation with Cu-Kα rays (wavelength λ = 1.54 angstrom). In the powder X-ray diffraction pattern, the ordinate axis indicates a diffraction intensity in a unit of count/second (cps), and the abscissa axis indicates a value of diffraction angle 2θ.
[Fig. 2] Fig. 2 shows a powder X-ray diffraction pattern of the B-form crystal prepared in Example 2, obtained by irradiation with Cu-Kα rays (wavelength λ = 1.54 angstrom). In the powder X-ray diffraction pattern, the ordinate axis of ordinate indicates a diffraction intensity in a unit of count/second (cps), and the abscissa axis indicates a value of diffraction angle 2θ.
[Fig. 3] Fig. 3 shows a powder X-ray diffraction pattern of the B-form crystal prepared in Example 3, obtained by irradiation with a Cu-Kα rays (wavelength λ = 1.54 angstrom). In the powder X-ray diffraction pattern, the ordinate axis indicates a diffraction intensity in a unit of count/second (cps), and the abscissa axis indicates a value of diffraction angle 2θ.

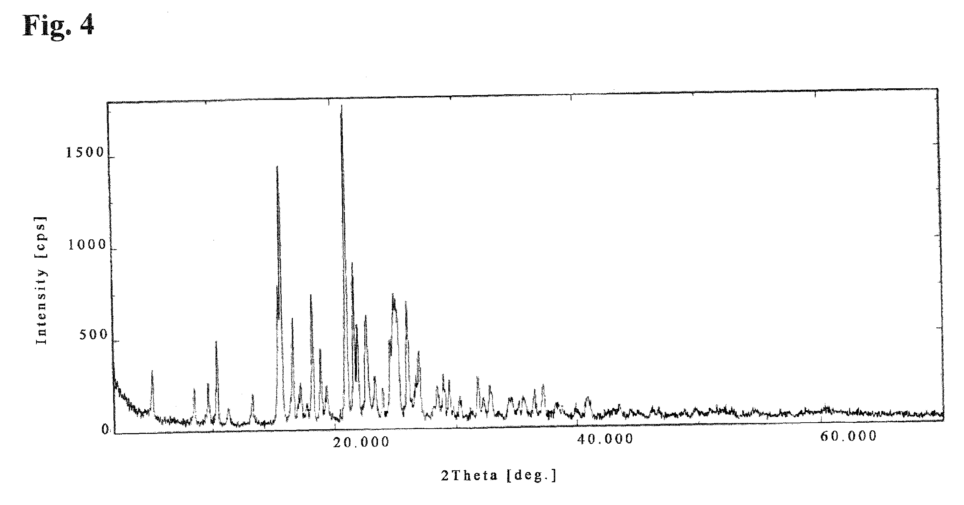

[Fig. 4] Fig. 4 shows a powder X-ray diffraction pattern of the A-form crystal prepared in Example 4, obtained by irradiation with a Cu-Kα rays (wavelength λ = 1.54 angstrom). In the powder X-ray diffraction pattern, the ordinate axis indicates a diffraction intensity in a unit of count/second (cps), and the abscissa axis indicates a value of diffraction angle 2θ.

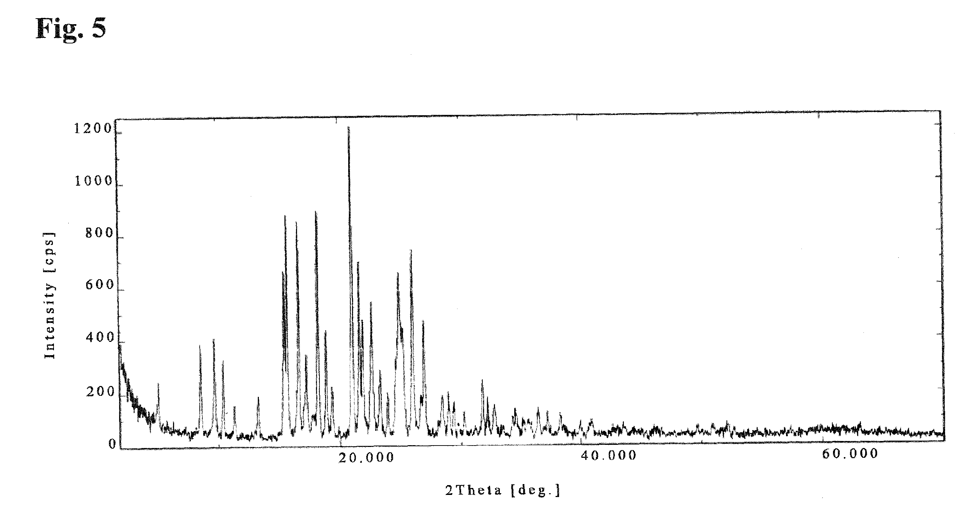

[Fig. 5] Fig. 5 shows a powder X-ray diffraction pattern of the A-form crystal prepared in Example 5, obtained by irradiation with a Cu-Kα rays (wavelength λ = 1.54 angstrom). In the powder X-ray diffraction pattern, the ordinate axis indicates a diffraction intensity in a unit of count/second (cps), and the abscissa axis indicates a value of diffraction angle 2θ.

### BEST MODE FOR CARRYING OUT THE INVENTION

The A-form crystal and B-form crystal of Compound (I) of the present invention, as described later in the Examples, can be prepared by the method described below. Specifically, the A-form crystal and B-form crystal can be obtained by:
(i) preparation by formation of 4-(2-aminopyridin-4-yl)-3-(4-fluorophenyl)-1-(1,4,5,6-tetrahydro-6-oxopyridazin-3-yl)-1H-pyrazole methanesulfonate from a free form thereof, and subsequent crystallization,
(ii) preparation by recrystallization of Compound (I), or
(iii) preparation by polymorphic transition of crystal of Compound (I).

### (i) Preparation by formation of methanesulfonate and subsequent crystallization

An A-form crystal of Compound (I) can be prepared by reacting 4-(2-aminopyridin-4-yl)-3-(4-fluorophenyl)-1-(1,4,5,6-tetrahydro-6-oxopyridazin-3-yl)-1H-pyrazole, which is a free form, with methanesulfonic acid in a good solvent, and then adding a poor solvent portionwise and stirring the resultant mixture while cooling. The portionwise addition of a poor solvent and the stirring of the mixture while cooling can be preformed by, for example, a method in which methanesulfonic acid is added dropwise or added, and then a part of a requisite amount of a poor solvent is added dropwise or added and the resultant mixture is stirred until a crystal starts precipitating, and then, after stirring the mixture for a certain period of time while cooling, the remaining part of the poor solvent is dropped or added to the mixture.

The "good solvent" of the present invention may be any solvent which does not inhibit the reaction and easily dissolves Compound (I), specifically including but not limited to alcohols such as methanol, ethanol, and 2,2,2-trifluoroethanol; amides such as N,N-dimethylformamide, dimethylacetamide, and 1-methyl-2-pyrrolidone; carboxylic acids such as acetic acid and propionic acid; nitriles such as acetonitrile and propionitrile; sulfur-containing solvents such as dimethyl sulfoxide; and water. Preferred are methanol, 2,2,2-trifluoroethanol, N,N-dimethylformamide, acetic acid, dimethyl sulfoxide, and water, and especially preferred are 2,2,2-trifluoroethanol, acetic acid, dimethyl sulfoxide, and water.
These good solvents may be used alone or in combination with one another.

The "poor solvent" of the present invention may be any solvent which does not inhibit the reaction and poorly dissolves Compound (I), specifically including but not limited to aliphatic hydrocarbons such as hexane, cyclohexane, heptane, ligroin, and petroleum ether; aromatic hydrocarbons such as benzene, toluene, and xylene; halogenated hydrocarbons such as dichloromethane, chloroform, carbon tetrachloride, 1,2-dichloroethane, chlorobenzene, and dichlorobenzene; ethers such as diethyl ether, diisopropyl ether, tert-butyl methyl ether, tetrahydrofuran, dioxane, dimethoxyethane, and diethylene glycol dimethyl ether; alcohols such as 2-propanol and 1-butanol; ketones such as acetone, methyl ethyl ketone, and diethyl ketone; and esters such as ethyl acetate, propyl acetate, and butyl acetate. Preferred are toluene, diisopropyl ether, tert-butyl methyl ether, 2-propanol, acetone, and ethyl acetate, and especially preferred are tert-butyl methyl ether, 2-propanol, acetone, and ethyl acetate.
These poor solvents may be used alone or in combination with one another.

The amount of the good solvent used in the preparation by formation of a methanesulfonate and subsequent crystallization varies according to the type of the solvent used, and is preferably 1 to 100 ml, further preferably 2 to 50 ml, especially preferably 3 to 20 ml based on 1 g of 4-(2-aminopyridin-4-yl)-3-(4-fluorophenyl)-1-(1,4,5,6-tetrahydro-6-oxopyridazin-3-yl)-1H-pyrazole which corresponds to the free form of Compound (I).

The amount of the methanesulfonic acid used is, preferably 0.5 to 5.0 mol, further preferably 0.9 to 2.0 mol, most preferably 0.95 to 1.5 mol based on 1 mol of the free form of Compound (I). The temperature for dropping or adding methanesulfonic acid varies according to the type or amount of the solvent used, and is generally -20 to 100°C, preferably 0 to 90°C, further preferably 10 to 80°C, most preferably 20 to 75°C.

The portionwise addition of the poor solvent may be appropriately performed by dividing the requisite amount of the poor solvent into at least two portions, and adding each of them at an arbitrary point in time, but it is preferred that the first addition is performed after dropping or adding methanesulfonic acid and before stirring the mixture while cooling. The amount of the poor solvent dropped or added as a part of the requisite amount (the amount of the poor solvent first added) before stirring the mixture while cooling varies according to the type or amount of the solvent used, and is preferably less than or equal to 10 times the amount of good solvent (volume ratio), further preferably less than or equal to 5 times (volume ratio). For example, when the good solvent is dimethyl sulfoxide and the poor solvent is ethyl acetate, the amount of the poor solvent is preferably less than or equal to 10 times the amount of good solvent (volume ratio), further preferably less than or equal to 3 times (volume ratio).

The temperature for dropping or adding the poor solvent on the first addition varies according to the type or amount of the solvent used, and is typically performed at the temperature for dropping or adding methanesulfonic acid. Such a temperature is generally -20 to 100°C, preferably 0 to 90°C, further preferably 10 to 80°C, most preferably 20 to 60°C.

The temperature for stirring the mixture while cooling varies according to the type or amount of the solvent used, and is generally -80 to 40°C, preferably -20 to 40°C, further preferably -10 to 30°C, most preferably 0 to 25°C.

With respect to the portionwise addition of the poor solvent, it is preferred that at least one further addition of the poor solvent is performed after stirring the mixture while cooling. The amount of the poor solvent dropped or added as the remaining part of the requisite amount varies according to the type or amount of the solvent used, and is preferably less than or equal to 20 times the amount of good solvent (volume ratio), further preferably less than or equal to 10 times (volume ratio). For example, when the good solvent is dimethyl sulfoxide and the poor solvent is ethyl acetate, the amount of the poor solvent is preferably less than or equal to 20 times the amount of good solvent (volume ratio), further preferably less than or equal to 5 times (volume ratio).

The temperature for the further dropped or added of the poor solvent after stirring the mixture while cooling varies according to the type or amount of the solvent used, and is typically performed at the temperature for stirring the mixture while cooling. Such a temperature is generally -80 to 40°C, preferably -20 to 40°C, further preferably -10 to 30°C, most preferably 0 to 25°C.

The time which takes to complete the drop or addition of the poor solvent used varies according to the type or amount of the solvent or the reaction temperature, and is generally 5 minutes to 48 hours, preferably 10 minutes to 24 hours.

The volume ratio of the good solvent used to the poor solvent used (requisite amount) is preferably from 19:1 to 1:19, further preferably from 9:1 to 1:9, especially preferably from 5:1 to 1:5.

The amount of the solvents used (the total amount of the good solvent and poor solvent) is, preferably 1 to 100 ml, further preferably 2 to 50 ml, especially preferably 5 to 20 ml base on 1 g of the free form of Compound (I).

### (ii) Preparation by recrystallization of compound (I)

When obtaining an A-form crystal or a B-form crystal by recrystallization, it is effective that a seed crystal of the same crystal form as that of a desired crystal is added. The amount of the seed crystal is, generally in the range of from 0.0001 to 0.2 g, preferably 0.00 1 to 0.1 g based on 1 g of Compound (I). It is preferred that the seed crystal is preliminarily finely ground.
The concentration of the solution of compound (I) to which a seed crystal is added is preferably in the supersaturated region of the crystal to be obtained.

The recrystallization is performed using a good solvent. The good solvent used in the recrystallization may be any solvent which does not inhibit the reaction and easily dissolves Compound (I), and specific examples and preferred examples of the good solvents are the same as those mentioned above.

The recrystallization is preferably performed using a poor solvent with using the above-mentioned good solvent. The poor solvent used in the recrystallization may be any solvent which does not inhibit the reaction and poorly dissolves Compound (I), and specific examples and preferred examples of the poor solvents are the same as those mentioned above.

In the recrystallization, the crystallization temperature varies according to the reagent and solvent, and is generally -20 to 100°C. In the case of an A-form crystal, the crystallization temperature is preferably 0 to 90°C, further preferably 10 to 80°C, most preferably 20 to &0°C.
In the case of a B-form crystal, the crystallization temperature is preferably 0 to 90°C, further preferably 10 to 80°C, most preferably 40 to 80°C.

In the recrystallization, the time which takes to complete the drop or addition of the poor solvent varies according to the type or amount of the solvent used, the reaction temperature and the like, and is generally 5 minutes to 48 hours, preferably 10 minutes to 24 hours.

In the recrystallization, specific examples of preferred combinations of the good solvent and poor solvent used for obtaining an A-form crystal and a B-form crystal of Compound (I) include a combination of water and 2-propanol, a combination of 2,2,2-trifluoroethanol and ethyl acetate, a combination of acetic acid and tert-butyl methyl ether, a combination of dimethyl sulfoxide and acetone, and a combination of dimethyl sulfoxide and ethyl acetate. An example of solvents used alone is acetic acid.

The volume ratio of the good solvent to the poor solvent used in the recrystallization is preferably from 19:1 to 1:19, further preferably from 9:1 to 1:9, especially preferably 5:1 to 1:5.

The total amount of the solvents used in the recrystallization is preferably 1 to 100 ml, further preferably 2 to 50 ml, especially preferably 5 to 20 ml based on 1 g of Compound (I).

The solvent used in the recrystallization may contain water. The water content of the solvent varies according to the type of the solvent, and is in the range of from 0 to a content such that water is saturated in the solvent. Specifically, for example, when the solvent is ethyl acetate, the water content is 0 to 0.033 g per 1 g of ethyl acetate.

### (iii) Preparation by polymorphic transition of crystal of Compound (I)

The B-form crystal and A-form crystal can also be prepared by polymorphic transition of crystal from the A-form crystal and B-form crystal, respectively.

In the present specification, the polymorphic transition of crystal includes solid phase transition in which transition of the crystal form occurs in the state of solid, and solvent-mediated phase transition in which transition occurs through a solvent. That is, the polymorphic transition of crystal may be performed either in the presence of a single solvent or a mixed solvent comprising of two or more solvents or in the absence of a solvent.

The heating temperature for obtaining a B-form crystal from an A-form crystal of Compound (I) by solid phase transition among the polymorphic transition of crystal is preferably 260 to 290°C, further preferably 270 to 280°C.

An example of the solvent used for obtaining an A-form crystal from a B-form crystal of Compound (I) by solvent-mediated phase transition among the polymorphic transition of crystal includes a good solvent. The good solvent used in the solvent-mediated phase transition may be any solvent which does not inhibit the reaction and easily dissolves Compound (I), and specific examples and preferred examples of the good solvents are the same as those mentioned above. The solvent-mediated phase transition is preferably performed using a mixed solvent of a good solvent and a poor solvent. The poor solvent used in the solvent-mediated phase transition may be any solvent which does not inhibit the reaction and poorly dissolves Compound (I), and specific examples and preferred examples of the poor solvents are the same as those mentioned above. An especially preferred example includes a mixed solvent of dimethyl sulfoxide and ethyl acetate.

In the solvent-mediated phase transition, the volume ratio of the good solvent to the poor solvent, for example, the volume ratio of dimethyl sulfoxide to ethyl acetate, is preferably from 19:1 to 1:19, further preferably from 9:1 to 1:9, especially preferably from 5:1 to 1:2.

The total amount of the solvents used in the solvent-mediated phase transition is, preferably 1 to 100 ml, further preferably 2 to 50 ml, especially preferably 5 to 20 ml based on 1 g of Compound (I). The solvent may contain water. The water content of the solvent varies according to the type of the solvent, and is in the range of from 0 to a content such that water is saturated in the solvent. Specifically, for example, when the solvent is ethyl acetate, the water content is 0 to 0.033 g per 1 g of ethyl acetate.

In the recrystallization and/or solvent-mediated phase transition, the crude crystal form of Compound (I) used for preparing a substantially pure A-form crystal or B-form crystal of Compound (I) may be an A-form crystal, a B-form crystal, or a mixture thereof, and may contain an amorphous form.
The crystals obtained by the methods of the present invention as described in detail in items (i) to (iii) above may be appropriately dried at room temperature or under appropriately warming or heating conditions and, if necessary, under reduced pressure or atmospheric pressure.

Compound (I) of the present invention has, as described in Patent document 1, an inhibitory action of p38MAP kinase and excellent suppressive effects on inflammatory cytokine production (particularly, TNF-α or IL-6 production) based thereon, and exhibits excellent oral absorption and low toxicity as well as excellent stability as a pharmaceutical product, and therefore is useful as an active ingredient of a pharmaceutical composition, especially as an active ingredient of a prophylactic or therapeutic agent for diseases in which inflammatory cytokines are implicated, such as rheumatic disease.

Examples of administrations of the crystal of Compound (I) of the present invention include an oral administration in the form of solid dosage forms such as a tablet, a capsule, a granule, or powder, and a parenteral administration in the form of suppository or the like. These pharmaceutical compositions are produced by a conventionally known method using an additive(s), such as an excipient, a lubricant, a binder, a disintegrating agent, a stabilizing agent, and/or a flavoring agent.

Examples of excipients include sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, low-substituted hydroxypropylcellulose, hydroxypropyl methylcellulose, carboxymethylcellulose, calcium carboxymethylcellulose, and internally crosslinked sodium carboxymethylcellulose; gum arabic; dextran; pullulan; silicate derivatives such as soft silicic anhydride, synthetic aluminum silicate, calcium silicate, and magnesium metasilicate aluminate; phosphate derivatives such as calcium hydrogenphosphate; carbonate derivatives, such as calcium carbonate; and sulfate derivatives such as calcium sulfate.

Examples of lubricants include talc; stearic acid; metal stearates such as calcium stearate and magnesium stearate; colloidal silica; waxes such as bee gum and spermaceti; boric acid; glycol; DL-leucine; carboxylic acids such as fumaric acid and adipic acid; sodium carboxylates such as sodium benzoate; sulfates such as sodium sulfate; laurylsulfates such as sodium laurylsulfate and magnesium laurylsulfate; sulfates such as sodium sulfate; silicic acids such as silicic anhydride and silicic acid hydrate; and starch derivatives mentioned above in the connection with the examples of excipients.

Examples of binders include the above-mentioned excipients; gelatin; polyvinylpyrrolidone; and polyethylene glycol. Examples of disintegrating agents include the above-mentioned excipients; chemically modified starch or cellulose derivatives such as croscarmellose sodium and carboxymethyl starch; and crosslinked polyvinylpyrrolidone. Examples of stabilizing agents include p-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; acetic anhydride; and sorbic acid. Examples of flavoring agents include sweetening agents, acidulants, and perfumes generally used.

The dosage amount of the crystal of Compound (I) of the present invention varies depending on the condition and age of a patient, the administration routes, and others, but, for example, in the case of an oral administration, it is desired that the amount between 0.1 mg (preferably 0.5 mg) as the lower limit and 2,000 mg (preferably 500 mg) as the upper limit per day is administered to an adult all at once or in several divided doses according to the condition of the patient.

### Examples

The present invention is described in more detail below with reference to the following Examples. However the invention is not intended to be limited thereto.

### (Example 1)

### Preparation of A-form crystal of 4-(2-aminopyridin-4-yl)-3-(4-fluorophenyl)-1-(1,4,5,6-tetrahydro-6-oxopyridazin-3-yl)-1H-pyrazole methanesulfonate

To a solution of 1.40 g (4.00 mmol) of 4-(2-aminopyridin-4-yl)-3-(4-fluorophenyl)-1-(1,4,5,6-tetrahydro-6-oxopyridazin-3-yl)-1H-pyrazole, which was obtained in the same manner as in Example 50 described in the WO2004/029043 pamphlet, in 6 ml of dimethyl sulfoxide was added 285 µl (4.40 mmol) of methanesulfonic acid at 45°C over 4 minutes, followed by stirring at the same temperature for 30 minutes. Then, 6 ml of ethyl acetate was added to the mixture over 50 minutes, and then the resultant mixture was cooled to 20°C and stirred for 24 hours. 12 ml of ethyl acetate was further added at the same cooling temperature over 40 minutes, followed by stirring for 10 minutes. Precipitated crystals were collected by filtration, and washed with 100 ml of ethyl acetate, and then dried under reduced pressure at 60°C for one hour to afford 1.52 g (yield: 85%) of an A-form crystal of 4-(2-aminopyridin-4-yl)-3-(4-fluorophenyl)-1-(1,4,5,6-tetrahydro-6-oxopyridazin-3-yl)-1H-pyrazole methanesulfonate.
A powder X-ray diffraction pattern of the A-form crystal obtained in Example 1, obtained by irradiation with Cu-Kα rays (wavelength λ = 1.54 angstrom) is shown in Fig. 1.

### (Example 2)

### Preparation of B-form crystal of 4-(2-aminopyridin-4-yl)-3-(4-fluorophenyl)-1-(1,4,5,6-tetrahydro-6-oxopyridazin-3-yl)-1H-pyrazole methanesulfonate

100 mg (0.22 mmol) of the A-form crystal of 4-(2-aminopyridin-4-yl)-3-(4-fluorophenyl)-1-(1,4,5,6-tetrahydro-6-oxopyridazin-3-yl)-1H-pyrazole methanesulfonate, which was obtained in the same manner as in Example 1, was heated in an electric furnace (KBF848N; manufactured by Koyo Thermo System Co., Ltd.) at 270 to 280°C for 10 minutes to afford 99 mg (yield: 99%) of a B-form crystal of 4-(2-aminopyridin-4-yl)-3-(4-fluorophenyl)-1-(1,4,5,6-tetrahydro-6-oxopyridazin-3-yl)-1H-pyrazole methanesulfonate.
A powder X-ray diffraction pattern of the B-form crystal obtained in Example 2, obtained by irradiation with Cu-Kα rays (wavelength λ = 1.54 angstrom) is shown in Fig. 2.

### (Example 3)

### Preparation of B-form crystal of 4-(2-aminopyridin-4-yl)-3-(4-fluorophenyl)-1-(1,4,5,6-tetrahydro-6-oxopyridazin-3-yl)-1H-pyrazole methanesulfonate

To a solution of 1.12 g (2.50 mmol) of 4-(2-aminopyridin-4-yl)-3-(4-fluorophenyl)-1-(1,4,5,6-tetrahydro-6-oxopyridazin-3-yl)-1H-pyrazole methanesulfonate, which was obtained in the same manner as in Example 80 described in the WO2004/0290 pamphlet, in 3.75 ml of dimethyl sulfoxide was added 1.88 ml of ethyl acetate at 75°C over 5 minutes. Then, 15 mg of the B-form crystal obtained in Example 2 was added as a seed crystal to the resultant mixture, and 9.37 ml of ethyl acetate was added thereto over 25 minutes. After stirring for 10 minutes, precipitated crystals were collected by filtration, and washed with 40 ml of ethyl acetate, and then dried under reduced pressure at 60°C for one hour to afford 954 mg (yield: 85%) of a B-form crystal of 4-(2-aminopyridin-4-yl)-3-(4-fluorophenyl)-1-(1,4,5,6-tetrahydro-6-oxopyridazin-3-yl)-1H-pyrazole methanesulfonate.
A powder X-ray diffraction pattern of the B-form crystal obtained in Example 3, obtained by irradiation with Cu-Kα rays (wavelength λ = 1.54 angstrom) is shown in Fig. 3.

### (Example 4)

### Preparation of A-form crystal of 4-(2-aminopyridin-4-yl)-3-(4-fluorophenyl)-1-(1,4,5,6-tetrahydro-6-oxopyridazin-3-yl)-1H-pyrazole methanesulfonate

To a solution of 670 mg (1.50 mmol) of the B-form crystal of 4-(2-aminopyridin-4-yl)-3-(4-fluorophenyl)-1-(1,4,5,6-tetrahydro-6-oxopyridazin-3-yl)-1H-pyrazole methanesulfonate, which was obtained in the same manner as in Example 3, in 2.25 ml of dimethyl sulfoxide was added 1.13 ml of ethyl acetate at 45°C over 10 minutes. Then, 6.7 mg of the A-form crystal obtained in Example 1 was added as a seed crystal to the resultant mixture, and 5.63 ml of ethyl acetate was added thereto over 50 minutes. After stirring for 10 minutes, precipitated crystals were collected by filtration, and washed with 20 ml of ethyl acetate, and then dried under reduced pressure at 60°C for one hour to afford 553 mg (yield: 83%) of an A-form crystal of 4-(2-aminopyridin-4-yl)-3-(4-fluorophenyl)-1-(1,4,5,6-tetrahydro-6-oxopyridazin-3-yl)-1H-pyrazole methanesulfonate.
A powder X-ray diffraction pattern of the A-form crystal obtained in Example 4, obtained by irradiation with Cu-Kα rays (wavelength λ = 1.54 angstrom) is shown in Fig. 4.

### (Example 5)

### Preparation of A-form crystal of 4-(2-aminopyridin-4-yl)-3-(4-fluorophenyl)-1-(1,4,5,6-tetrahydro-6-oxopyridazin-3-yl)-1H-pyrazole methanesulfonate

To 200 mg (0.448 mmol) of the B-form crystal of 4-(2-aminopyridin-4-yl)-3-(4-fluorophenyl)-1-(1,4,5,6-tetrahydro-6-oxopyridazin-3-yl)-1H-pyrazole methanesulfonate, which was obtained in the same manner as in Example 3, was added 2 ml of a mixed solvent of dimethyl sulfoxide-ethyl acetate {1:1 (volume ratio)}, and the resultant suspension was stirred at 75°C for one hour. After cooling to 45°C and stirring at the same temperature for one hour, 5 mg of the A-form crystal obtained in Example 1 was added as a seed crystal thereto. Further after cooling to 25°C and stirring at the same temperature for 66 hours, precipitated crystals were collected by filtration, and washed with 10 ml of ethyl acetate, and then dried under reduced pressure at 60°C for one hour to afford 130 mg (yield: 65%) of an A-form crystal of 4-(2-aminopyridin-4-yl)-3-(4-fluorophenyl)-1-(1,4,5,6-tetrahydro-6-oxopyridazin-3-yl)-1H-pyrazole methanesulfonate.
A powder X-ray diffraction pattern of the A-form crystal obtained in Example 5, obtained by irradiation with Cu-Kα rays (wavelength λ = 1.54 angstrom) is shown in Fig. 5.

### (Comparative Example 1)

To a solution of 350 mg (1.00 mmol) of 4-(2-aminopyridin-4-yl)-3-(4-fluorophenyl)-1-(1,4,5,6-tetrahydro-6-oxopyridazin-3-yl)-1H-pyrazole, which was obtained in the same manner as in Example 50 described in the WO2004/02904 pamphlet, in 1.5 ml of dimethyl sulfoxide was added 71.5 µl (1.10 mmol) of methanesulfonic acid at 45°C over 30 minutes, followed by stirring at the same temperature for 30 minutes. Then, 4.5 ml of ethyl acetate was added thereto over 30 minutes, followed by stirring for another 5 minutes. Precipitated crystals were collected by filtration, and washed with 30 ml of ethyl acetate, and then dried under reduced pressure at 60°C for one hour to afford 339 mg (yield: 76%) of 4-(2-aminopyridin-4-yl)-3-(4-fluorophenyl)-1-(1,4,5,6-tetrahydro-6-oxopyridazin-3-yl)-1H-pyrazole methanesulfonate. From the powder X-ray diffraction pattern, it was found that the obtained crystals were a mixture of the A-form crystal and B-form crystal.

### Preparation Examples

### (Preparation Example 1) (Hard capsule)

50 mg of the A-form crystal of Compound (I) in Example 1, 128.7 mg of lactose, 70 mg of cellulose, and 1.3 mg of magnesium stearate are mixed and passed through a 60 mesh sieve, followed by placing 250 mg of the powder in a No. 3 gelatin capsule to obtain a capsule preparation.

### (Preparation Example 2) (Tablet)

50 mg of the A-form crystal of Compound (I) in Example 1, 124 mg of lactose, 25 mg of cellulose, and 1 mg of magnesium stearate are mixed and formed into a tablet with tableting machine to obtain a tablet preparation weighing 200 mg of the mixture per tablet. This tablet preparation can be provided with a sugar-coat as necessary.

### INDUSTRIAL APPLICABILITY

The present invention presents a substantially pure A-form crystal and B-form crystal of 4-(2-aminopyridin-4-yl)-3-(4-fluorophenyl)-1-(1,4,5,6-tetrahydro-6-oxopyridazin-3-yl)-1H-pyrazole methanesulfonate, which have prophylactic or therapeutic effect on chronic rheumatoid arthritis based on suppressive effects on cytokine production, and methods for their preparations.

## Claims

1. A substantially pure crystal of 4-(2-aminopyridin-4-yl)-3-(4-fluorophenyl)-1-(1,4,5,6-tetrahydro-6-oxopyridazin-3-yl)-1H-pyrazole methanesulfonate.

2. The crystal (A-form crystal) according to claim 1, which shows main d spacings peaks at 8.5, 5.7, 4.9, and 4.2 angstroms in a powder X-ray diffraction pattern, obtained by irradiation with Cu-Kα rays..

3. The crystal (A-form crystal) according to claim 1, which shows diffraction angles 2θ at 10.4±0.2, 15.7±0.2, 18.2±0.2, and 21.0±0.2 (degrees) in a powder X-ray diffraction pattern obtained by irradiation with Cu-Kα rays.

4. A method for preparing the A-form crystal according to claim 2 or 3, which comprises reacting 4-(2-aminopyridin-4-yl)-3-(4-fluorophenyl)-1-(1,4,5,6-tetrahydro-6-oxopyridazin-3-yl)-1H-pyrazole with methanesulfonic acid in a good solvent, and then adding a poor solvent portionwise and stirring the resultant mixture while cooling.

5. The method according to claim 4, wherein the temperature at the start of the first addition of the poor solvent in the portionwise addition of the poor solvent is 20 to 60°C.

6. The method according to claim 4, wherein the temperature for the stirring of the mixture while cooling is 0 to 25°C.

7. The method according to claim 4, which comprises, after stirring the mixture while cooling, further dropping or adding the poor solvent.

8. The method according to claim 4, wherein the good solvent is dimethyl sulfoxide.

9. The method according to claim 4, wherein the poor solvent is ethyl acetate.

10. A method for preparing the A-form crystal according to claim 2 or 3, which comprises recrystallizing 4-(2-aminopyridin-4-yl)-3-(4-fluorophenyl)-1-(1,4,5,6-tetrahydro-6-oxopyridazin-3-yl)-1H-pyrazole methanesulfonate by dissolving in a good solvent, and then adding a poor solvent and a seed crystal of the A-form crystal.

11. The method according to claim 10, wherein the crystallization temperature in the recrystallization is 20 to 60°C.

12. The method according to claim 10, wherein the good solvent is dimethyl sulfoxide.

13. The method according to claim 10, wherein the poor solvent is ethyl acetate.

14. The crystal (B-form crystal) according to claim 1, which shows main d spacings peaks at 10.9, 5.4, 5.2, and 4.7 angstroms in a powder X-ray diffraction pattern obtained by irradiation with Cu-Kα rays.

15. The crystal (B-form crystal) according to claim 1, which shows diffraction angles 2θ at 8.1±0.2, 16.3±0.2, 17.0±0.2, and 18.9±0.2 (degrees) in a powder X-ray diffraction pattern obtained by irradiation with Cu-Kα rays.

16. A method for preparing the B-form crystal according to claim 14 or 15, which comprises performing solid phase transition from an A-form crystal of 4-(2-aminopyridin-4-yl)-3-(4-fluoroplaenyl)-1-(1,4,5,6-tetrahydro-6-oxopyridazin-3-yl)-1H-pyrazole methanesulfonate.

17. The method according to claim 16, wherein the solid phase transition is performed at 270 to 280°C.

18. A method for preparing the B-form crystal according to claim 14 or 15, which comprises recrystallizing 4-(2-aminopyridin-4-yl)-3-(4-fluorophenyl)-1-(1,4,5,6-tetrahydro-6-oxopyridazin-3-yl)-1H-pyrazole methanesulfonate by dissolving in a good solvent, and then adding a poor solvent and a seed crystal of the B-form crystal.

19. The method according to claim 18, wherein the crystallization temperature in the recrystallization is 40 to 80°C.

20. The method according to claim 18, wherein the good solvent is dimethyl sulfoxide.

21. The method according to claim 18, wherein the poor solvent is ethyl acetate.

22. A method for preparing the A-form crystal according to claim 2 or 3, which comprises performing solvent-mediated phase transition from a B-form crystal of 4-(2-aminopyridin-4-yl)-3-(4-fluorophenyl)-1-(1,4,5,6-tetrahydro-6-oxopyridazin-3-yl)-1H-pyrazole methanesulfonate.

23. The method according to claim 22, wherein the solvent used in the solvent-mediated phase transition is a mixed solvent of dimethyl sulfoxide and ethyl acetate.

24. The method according to claim 22, which comprises adding a seed crystal of the A-form crystal.

25. A pharmaceutical composition comprising the crystal of 4-(2-aminopyridin-4-yl)-3-(4-fluorophenyl)-1-(1,4,5,6-tetrahydro-6-oxopyridazin-3-yl)- 1H-pyrazole methanesulfonate according to any one of claims 1, 2, 3, 14 and 15 as an active ingredient.

26. A p38MAP kinase inhibitor comprising the crystal of 4-(2-aminopyridin-4-yl)-3-(4-fluorophenyl)-1-(1,4,5,6-tetrahydro-6-oxopyridazin-3-yl)- 1H-pyrazole methanesulfonate according to any one of claims 1, 2, 3, 14 and 15 as an active ingredient.

27. A therapeutic agent for rheumatic diseases comprising the crystal of 4-(2-aminopyridin-4-yl)-3-(4-fluorophenyl)-1-(1,4,5,6-tetrahydro-6-oxopyridazin-3-yl)-1H-pyrazole methanesulfonate according to any one of claims 1, 2, 3, 14 and 15 as an active ingredient.
